(19) Europäisches Patentamt
European Patent Office
Office européen des brevets

(11) Veröffentlichungsnummer: **0 063 349**
**B1**

(12) **EUROPÄISCHE PATENTSCHRIFT**

(45) Veröffentlichungstag der Patentschrift: **03.10.84**

(51) Int. Cl.³: **C 07 D 263/44**, C 07 D 253/06 // C07C103/88

(21) Anmeldenummer: **82103111.9**

(22) Anmeldetag: **13.04.82**

(54) **5-Acyloxy-4 (5H)-oxazolonium-Salze, Verfahren zu ihrer Herstellung und ihre Verwendung als Zwischenprodukte zur Synthese von herbizid wirksamen Triazinonen.**

(30) Priorität: **22.04.81 DE 3115970**

(43) Veröffentlichungstag der Anmeldung: **27.10.82 Patentblatt 82/43**

(45) Bekanntmachung des Hinweises auf die Patenterteilung: **03.10.84 Patentblatt 84/40**

(84) Benannte Vertragsstaaten: **BE CH DE FR GB IT LI NL**

(56) Entgegenhaltungen:
**TETRAHEDRON LETTERS, Band 28, Nr. 15, August 1972, Seiten 4065-4074, Oxford, G.B., N. SUZUKI et al.: "Studies on firefly bioluminescence - I. Synthesis and spectral properties of firefly oxyluciferin.A possible emitting species in firefly bioluminescence"**
**DIE MAKROMOLEKULARE CHEMIE, Band 131, Nr. 3199, 17. Februar 1970, Seiten 247-257, Basel, CH., H. DIEFENBACH et al.: "Über ungesättigte Harnstoff- und Oxalsäurederivate"**
**CHEMICAL ABSTRACTS, Band 93, Nr. 1, 7. Juli 1980, Seite 740, Nr. 8064q, Columbus, Ohio, USA, V.G. KUL'NEVICH et al.: "Substituted 4(5H)oxazolones and their salts. 2. Synthesis of 2-(beta(alkyl-,aryl-,furyl)vinyl)-5-(1,1-dimethyl-2-acetoxyethyl)-4(5H)-oxazolium perchlorates"**

(73) Patentinhaber: **BAYER AG, Konzernverwaltung RP Patentabteilung, D-5090 Leverkusen 1 Bayerwerk (DE)**

(72) Erfinder: **Bonse, Gerhard, Dr., Wolfskaul 3, D-5000 Köln 80 (DE)**
Erfinder: **Marzolph, Gerhard, Dr., Roggendorfstrasse 65, D-5000 Köln 80 (DE)**
Erfinder: **Blank, Heinz Ulrich, Dr., Am Geusfelde 35, D-5068 Odenthal (DE)**

EP 0 063 349 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neuartige, in Form einer Lösung erhältliche 4(5H)-Oxazoloniumsalze mit einem Acyloxyrest in 5-Stellung, ein Verfahren zu ihrer Herstellung und ihre Verwendung als Zwischenprodukte zur Synthese von bekannten, herbizid wirksamen 3,4,6-trisubstituierten 1,2,4-Triazin-5(4H)-onen.

Es sind bereits 4(5H)-Oxazoloniumsalze bekannt, die in 5-Stellung Wasserstoff oder niedere Alkylgruppen tragen:

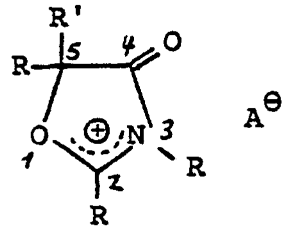

R=Niederalkyl;
R'=Wasserstoff oder Niederalkyl;
A=$ClO_4$, Cl, Br.

Diese Substanzen wurden hergestellt durch Umsetzung von α-Hydroxycarbonsäureamiden mit Anhydriden in Gegenwart von 70%iger Perchlorsäure („Khim. Geterosikl. Soedin", *1977*, 702;„Zh. Org. Khim.", *12* [1976] 1134 und SU-A Nr. 159825 [25.1.1979]) oder durch Umsetzung von α-Halogenessigsäurebromiden mit Amiden („Ukr. chim. Z.", *30* [1964] 3, 265) oder durch Umsetzung von Chloracetamiden mit Säurechloriden („Ukr. chim. Z.", *30* [1964] 6, 618 und *ibid.* *32* [1966] 2, 202).

Nach allen oben beschriebenen Verfahren können nur 4(5H)-Oxazoloniumsalze erhalten werden, die in 5-Stellung Wasserstoff oder Alkylgruppen tragen. 5-Acyloxy-4(5H)-oxazoloniumsalze sind auf diesen Wegen nicht zugänglich.

Es wurde nun überraschend gefunden, dass man die neuen 5-Acyloxy-4(5H)-oxazoloniumsalze der allgemeinen Formel I:

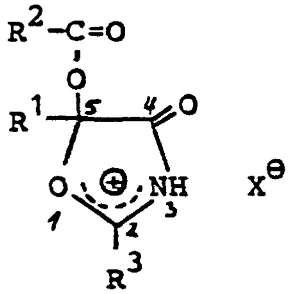

(I)

in welcher

$R^1$ für einen gegebenenfalls durch Alkoxy mit 1 bis 4 Kohlenstoffatomen, Carbalkoxy mit 1 bis 4 Kohlenstoffatomen in der Alkoxygruppe, Nitro, Cyano und/oder Halogen (wie z.B. Fluor, Chlor, Brom oder Jod) substituierten, geradkettigen oder verzweigten Alkylrest mit 1 bis 4 Kohlenstoffatomen; Carbonsäure für einen gegebenenfalls durch Alkyl, Alkoxy oder Carbalkoxy mit jeweils bis zu 4 Kohlenstoffatomen, Nitro, Cyano und/oder Halogen (wie z.B. Fluor, Chlor und Brom) substituierten Cycloalkylrest mit 3 bis 6 Kohlenstoffatomen im Ringsystem; für einen gegebenenfalls durch Alkyl, Alkoxy oder Carbalkoxy mit jeweils bis zu 4 Kohlenstoffatomen, Nitro und/oder Halogen (wie z.B. Fluor, Chlor und Brom) substituierten Phenyl- oder Naphthylrest; oder für einen gegebenenfalls durch Alkyl, Alkoxy oder Carbalkoxy mit jeweils bis zu 4 Kohlenstoffatomen, Nitro, Cyano und/oder Halogen (wie z.B. Fluor, Chlor und Brom) substituierten 5- oder 6gliedrigen heterocyclischen Rest steht, der 1 bis 3 Heteroatome wie Sauerstoff, Schwefel und/oder Stickstoff im Ring enthalten und ausserdem mit einem Benzolring annelliert sein kann,

$R^2$ und $R^3$ gleich oder verschieden sind und für einen gegebenenfalls chlorsubstituierten Alkylrest mit 1 bis 4 Kohlenstoffatomen oder für einen Phenylrest stehen, und

$X^{\ominus}$ für das Anion einer anorganischen oder organischen Säure mit einem $pK_s$-Wert kleiner als 2 steht, in Form einer Lösung erhält, wenn man Acylcyanide der allgemeinen Formel II:

$$R^1-CO-CN \qquad (II)$$

in welcher

$R^1$ die oben angegebene Bedeutung hat,
mit Carbonsäureanhydriden der allgemeinen Formel III:

$$R^2-CO-O-CO-R^3 \qquad (III)$$

in welcher

$R^2$ und $R^3$ gleich oder verschieden sind und die oben angegebene Bedeutung haben,
in Gegenwart einer anorganischen oder organischen Säure mit einem $pK_s$-Wert kleiner als 2 und gegebenenfalls in Gegenwart eines Lösungsmittels bei Temperaturen zwischen 0 und 120°C umsetzt.

Verwendet man Pivaloylcyanid und Essigsäureanhydrid als Ausgangsstoffe und führt die Umsetzung in Gegenwart von konzentrierter Schwefelsäure durch, so kann der Reaktionsablauf durch das folgende Formelschema wiedergegeben werden:

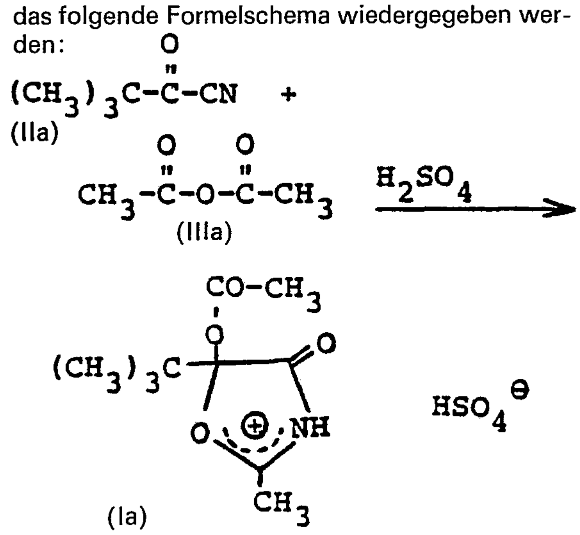


(IIa) (IIIa) (Ia)

Die als Ausgangsstoffe einzusetzenden Acylcyanide sind durch Formel II allgemein definiert. Als Beispiele für insbesondere in Frage kommende heterocyclische Reste $R^1$ seien genannt: Morpholinyl, Imidazolyl, Pyrazolyl, Pyrrolyl, Isoxazolyl, Piperidinyl, Oxazolyl, 1,2,4-Triazol-1-yl, 1,2,4-Triazol-4-yl, 1,2,3-Triazolyl, 1,2,4-Thiadiazol-2-yl, Benzimidazolyl und Furanyl.

Die Acylcyanide der Formel II sind zum Teil bekannt; noch nicht bekannte Acylcyanide können nach bekannten Verfahren hergestellt werden (vgl. „Angew. Chem.", *68*, S. 425-435 [1965]; ferner DE-A Nrn. 2614240, 2614241, 2614242, 2708182, 2708183).

Als im Rahmen dieser Erfindung besonders bevorzugte Acylcyanide seien Pivaloylcyanid und Benzoylcyanid genannt.

Die weiterhin als Ausgangsstoffe einzusetzenden Carbonsäureanhydride der Formel III sind zum Teil grosstechnisch verfügbar bzw. nach allgemein bekannten Methoden, z.B. aus den entsprechenden Carbonsäuren herstellbar. Dabei kann die Bildung der Carbonsäureanhydride der Formel III gegebenenfalls auch im Reaktionsmedium unter Verwendung an sich bekannter anhydridisierender Reagenzien, wie z.B. konzentrierter Schwefelsäure, erfolgen.

Im Rahmen dieser Erfindung sind besonders bevorzugte Carbonsäureanhydride Essigsäureanhydrid, Propionsäureanhydrid und die Anhydride der Chloressigsäuren.

Die erfindungsgemässe Umsetzung wird in Gegenwart einer Säure mit einem $pK_s$-Wert kleiner als 2 durchgeführt. Als solche Säuren kommen anorganische Säuren, wie konzentrierte Schwefelsäure, Halogenwasserstoffsäuren, z.B. wasserfreier Chlorwasserstoff und Bromwasserstoff, sowie Perchlorsäure und Phosphorsäure in Frage. Weiterhin geeignet sind aliphatische und aromatische Sulfon- und Phosphonsäuren sowie Halogenalkancarbonsäuren wie z.B. Trifluoressigsäure. Bevorzugt wird konzentrierte Schwefelsäure verwendet.

Es ist möglich, die erfindungsgemässe Umsetzung in Gegenwart einer oder mehrerer solcher Säuren durchzuführen.

Die Reaktionstemperaturen können in einem grösseren Bereich variiert werden und liegen zwischen 0 und 120, vorzugsweise zwischen 10 und 60°C.

Die Reaktion wird im allgemeinen bei Normaldruck durchgeführt.

Die Umsetzung kann in Abwesenheit oder in Gegenwart eines Lösungsmittels bzw. Lösungsvermittlers durchgeführt werden. Als Lösungsvermittler kommen bestimmte organische Lösungsmittel in Frage; besonders geeignet sind Eisessig und Dichlormethan, ferner Dialkylether, wie Diethyl- oder Diisopropylether.

Bei der Durchführung des erfindungsgemässen Verfahrens setzt man im allgemeinen auf 1 mol Acylcyanid der Formel II 0,5 bis 10, vorzugsweise 0,8 bis 4 mol Carbonsäureanhydrid der Formel III ein; besonders bevorzugt ist ein Molverhältnis Acylcyanid II zu Carbonsäureanhydrid III von 1:1 bis 1:2.

Die für die Durchführung des erfindungsgemässen Verfahrens erforderlichen Säuren werden in katalytischen bis überstöchiometrischen Mengen eingesetzt. Im allgemeinen setzt man auf 1 mol Acylcyanid II 0,5 bis 10, vorzugsweise 0,8 bis 8 mol, besonders bevorzugt 1 bis 4 mol Säure ein.

Besonders vorteilhaft ist ein Molverhältnis Carbonsäureanhydrid III zu Säure von 1:2.

Damit ergibt sich, dass ein Molverhältnis Acylcyanid II zu Oxazoloniumsalzeanhydrid III zu Säure von 1:1:2 bis 1:2:4 ganz besonders günstig ist.

Zweckmässigerweise geht man bei der Durchführung des Verfahrens so vor, dass man die Säure und das Anhydrid bzw. die Mischung aus Carbonsäure und anhydridisierendem Reagens, gegebenenfalls unter Zusatz eines Lösungsmittels, vorlegt und das Acylcyanid, gegebenenfalls in einem Lösungsmittel, zugibt.

Die Reaktionszeiten betragen im allgemeinen 1 bis 10 h.

Die nach dem oben beschriebenen Verfahren hergestellte Reaktionsmischung stellt eine Lösung der 5-Acyloxy-4(5H)-oxazoloniumsalze I dar.

Die Struktur der Oxazoloniumsalze geht eindeutig aus den IR-, $^{1}H$-NMR- und $^{13}C$-NMR-Spektren der Reaktionsmischung hervor. Dies sei am Beispiel des 5-Acetoxy-5-tert.-butyl-2-methyl-4(5H)-oxazoloniumions Ia dargestellt. Das Ion wird erhalten durch Vermischen von Pivaloylcyanid, Acetanhydrid und wasserfreier Schwefelsäure im molaren Verhältnis 1:1:3, entsprechend der oben beschriebenen Verfahrensweise.

Das IR-Spektrum dieser Lösung zeigt ausser den Banden der Schwefelsäure drei starke Signale. Diese Signale werden im Einklang mit den vergleichbaren Daten der bekannten 4(5H)-Oxazoloniumperchlorate folgendermassen zugeordnet (Tabelle 1):

Das Signal bei 1830 $cm^{-1}$ entstammt der Valenzschwingung der Carbonylgruppe im Oxazoloniumsystem. Sie ist durch die Nachbarschaft des positiven Fragments zu höheren Frequenzen verschoben. Das Signal bei 1785 $cm^{-1}$ fällt in den Absorptionsbereich von Estercarbonylgruppen, die neben elektronegativen Gruppen stehen, wie z.B. in Enolestern. Sie kann hier der 5-Acetoxygruppe zugeordnet werden. Die Signale zwischen 1590 und 1530 $cm^{-1}$ können im Einklang mit den Vergleichssubstanzen den Gerüstschwingungen des Oxazoloniumkerns zugeordnet werden.

*(Tabelle auf der nächsten Seite)*

Das Signal bei 1,30 ppm mit einem Integral entsprechend 9 Protonen kann zweifelsfrei den drei Methylgruppen der tert.-Butylgruppe zugeordnet werden. Das Signal bei 3,17 ppm entspricht einer Methylgruppe neben einem stark positivierten Kohlenstoffatom. Durch Vergleich mit den Literaturangaben kann dieses Signal der 2-Methylgruppe zugeordnet werden. Das Signal bei 2,85 ppm muss der 5-Acetoxygruppe zugeordnet werden. Es ist gegenüber der Acetoxygruppe der Vergleichssubstanz um 0,55 ppm zu tieferem Feld verschoben. Dies beruht auf zwei Effekten:

Die Acetoxygruppe in Ia ist an ein stark positiviertes Kohlenstoffatom gebunden, wodurch das Signal der $CH_3$-Gruppe einen Shift nach tieferem Feld erhält. Zum anderen verursacht die Schwefelsäure gegenüber der Trifluoressigsäure als Lösungsmittel durch partielle Protonierung der

*Tabelle 1*

*IR-Spektren der Oxazoloniumsalze ($\nu$: $cm^{-1}$)*

| | C=O neben 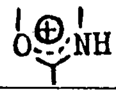 | $O-\overset{O}{\overset{\|}{C}}-CH_3$ neben positivierten C | $O-\overset{O}{\overset{\|}{C}}-CH_3$ normal | 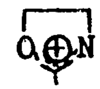 Gerüst-schwingung | Literatur |
|---|---|---|---|---|---|
| 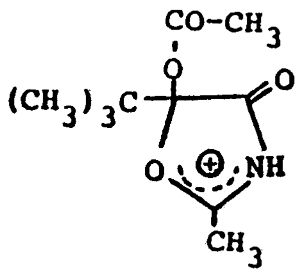 $HSO_4^{\ominus}$ (Ia) | 1830 | 1783 | — | 1590<br>1570(Sh),<br>1530(Sh) | — |
| 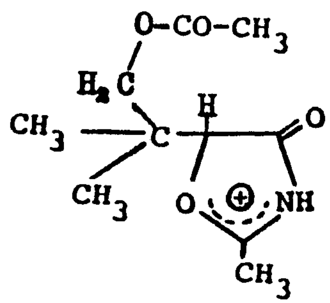 $ClO_4^{\ominus}$ | 1810 | — | 1720 | 1580, 1500 | „Zh. Org. Khim.“, *12* (1976) S. 1134 |
| 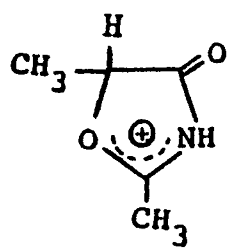 $ClO_4^{\ominus}$ | 1830 | — | — | 1590,<br>1548-1515 | „Khim. Geterosikl. Soedin“, *1977*, 702 |

Das $^{1}H$-NMR-Spektrum der Lösung von Ia zeigt drei Signale (Tabelle 2), die mit der Struktur des 4(5H)-Oxazoloniumsystems im Einklang stehen.

*Tabelle 2*

*$^{1}H$-NMR-Spektrum der 4(5H)-Oxazoloniumsalze (δ: ppm) gegen Tetramethylsilan (TMS) als internen Standard*

| | Signale | (Zuordnung) | Lösungsmittel |
|---|---|---|---|
| 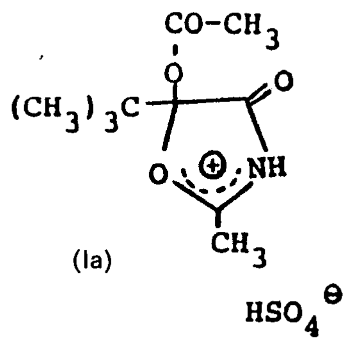 (Ia) | 1,30 ($3CH_3$) | 2,85 ($CH_3-C=O$)<br>3,17 ($2-CH_3$) | $H_2SO_4$ |
| 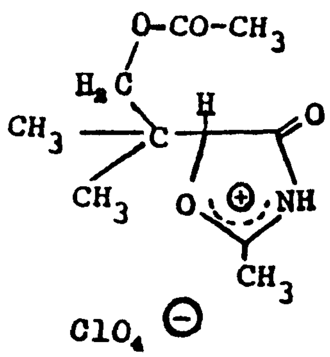 | 1,40 ($2CH_3$) | 2,30 ($CH_3CO$)<br>3,08 ($2-CH_3$)<br>4,20 ($CH_2$)<br>5,52 (5-CH) | $CF_3COOH$ |
| | (aus „Zh. Org. Khim.", *12,* [1976] 1134) | | |

Estercarbonylgruppe einen zusätzlichen Shift nach tieferem Feld.

Ein unabhängiger Strukturbeweis für die in der Reaktionsmischung vorliegenden 5-Acyloxy-4(5H)-oxazoloniumionen ergibt sich aus den $^{13}C$-NMR-Spektren. Es wurde ein Reaktionsgemisch aus Pivaloylcyanid, Acetanhydrid und Schwefelsäure im stöchiometrischen Verhältnis von 1:2:4 vermessen.

Ausser den beiden Signalen bei 189 und 19,5 ppm, die der protonierten Form der Essigsäure zugeordnet werden können, treten 8 weitere Signale auf Tabelle 3, die den 8 unterschiedlichen C-Atomen des 5-Acetoxy-5-tert.-butyl-4(5H)-oxazoloniumions Ia zugeordnet werden können.

Das Signal bei 193 ppm entstammt einem stark positivierten C-Atom, ähnlich dem Acetylkation. Es ist das 2-C-Atom des Oxazolinonsystems. Die Signale bei 171 und 167 ppm lassen sich zwanglos dem Carbonylkohlenstoffatom der 5-Acetoxygruppe und dem 4-C-Atom des Oxazoloniumsystems zuordnen. Das Signal bei 109 ppm entspricht in der Shiftlage einem C-Atom zwischen zwei Sauerstoffatomen, wie bei Ketalen, und wird hier dem 5-C-Atom des Oxazoloniumsystems zugeordnet. Das Signal bei 39,1 ppm entspricht dem tertiären C-Atom und das bei 22,7 ppm den primären C-Atomen der tert.-Butylgruppe. Die Signale bei 20,6 und 17,0 ppm entstammen den C-Atomen der Methylgruppen an C-2 des Oxazoloniumsystems und in der Acetylgruppe.

*(Tabelle auf der nächsten Seite)*

Die nach dem erfindungsgemässen Verfahren herstellbaren 5-Acyloxy-4(5H)-oxazoloniumsalze I sind neu und können als Zwischenprodukte zur Herstellung von bekannten, herbizid wirksamen Triazinonen (vgl. z.B. DE-A Nrn. 1542873, 1695596 und 1795784, ferner US-A Nrn. 3671523 und 3905801) verwendet werden.

Es wurde gefunden, dass man 1,2,4-Triazin-5(4H)-one der Formel IV:

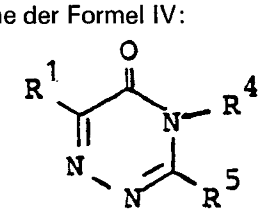 (IV)

in welcher

$R^1$ die oben angegebene Bedeutung hat,

*Tabelle 3*

*$^{13}C$-NMR-Spektren (δ: ppm)*

| Substanz | Lösungs-mittel | Zuordnung | | | | | | | | | |
|---|---|---|---|---|---|---|---|---|---|---|---|
| | | 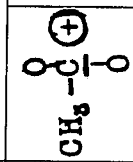 | 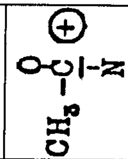 | 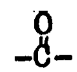 | 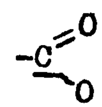 | 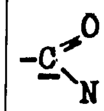 | -C≡N | 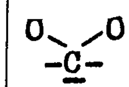 | $C_{tertiär}$ | 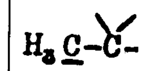 | 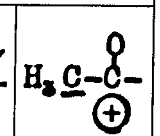 |
| 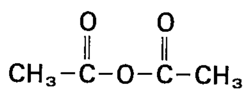 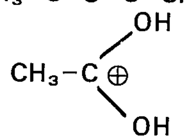 | $D_2SO_4$ | 189 | — | — | — | — | — | — | — | — | 19,5 |
| 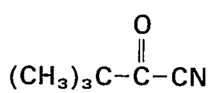 | $CDCl_3$ | — | — | 182 | — | — | 112 | — | 45 | 24,5 | — |
| 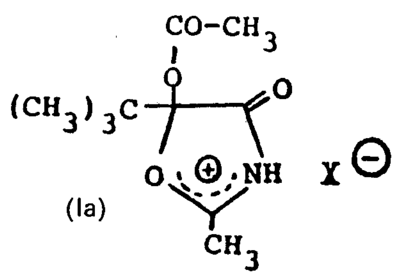 (Ia) | $D_2SO_4$ | — | 193 | — | 171 | 167 | — | 109 | 39,1 | 22,7 | 20,6<br>17,0 |

$R^4$ für Wasserstoff, Alkyl mit 1 bis 4 C-Atomen, Aryl mit 6 bis 10 C-Atomen, Amino, mono- oder disubstituierte Aminogruppen steht, und

$R^5$ für Wasserstoff, Alkyl mit 1 bis 4 C-Atomen, Aryl mit 6 bis 10 C-Atomen, Hydroxy, Alkoxy mit 1 bis 4 C-Atomen, Aryloxy mit 6 bis 10 C-Atomen, Mercapto, Alkylmercapto mit 1 bis 4 C-Atomen, Amino, mono- oder disubstituierte Aminogruppen steht,
in guten Ausbeuten erhält, wenn man 5-Acyloxy-4(5H)-oxazoloniumsalze I in Lösung entweder direkt oder nach vorheriger Verseifung zu den α-Ketocarbonsäure-N-acylamiden der Formel V:

$$R^1-CO-CO-NH-CO-R^3 \qquad (V)$$

in welcher

$R^1$ und $R^3$ die oben angegebene Bedeutung haben,
mit Hydrazinderivaten der Formel VI:

$$NH_2-N=\overset{\overset{\displaystyle R^5}{|}}{C}-NH-R^4 \qquad (VI)$$

in welcher

$R^4$ und $R^5$ die oben angegebene Bedeutung haben,
umsetzt.

Die erfindungsgemässen 5-Acyloxy-4(5H)-oxazoloniumsalze I stellen somit eine neue, wertvolle Klasse von Zwischenprodukten z.B. zur Synthese von α-Ketocarbonsäure-N-acylamiden (sowie – durch weitere Hydrolyse – von den entsprechenden, vielfältig verwendbaren α-Ketocarbonsäuren der Formel $R^1-CO-COOH$) und von herbizid wirksamen 1,2,4-Triazin-5-onderivaten dar.

Die α-Ketocarbonsäure-N-acylamide V als solche sind Gegenstand eines älteren Schutzbegehrens (vgl. EP-A Nr. 0035707).

Die Umsetzung dieser α-Ketocarbonsäure-N-acylamide V mit Thiocarbohydrazid (VI, $R^4=NH_2$, $R^5=SH$) zu Triazinonen (IV, $R^5=SH$) ist ebenfalls Gegenstand eines älteren Schutzbegehrens (vgl. EP-A Nr. 0035708).

Im Rahmen dieser Erfindung ist die direkte Umsetzung der neuen 5-Acyloxy-4(5H)-oxazoloniumsalze I mit den Hydrazinderivaten VI zu den gewünschten 1,2,4-Triazin-5(4H)-onderivaten IV bevorzugt gegenüber der Verfahrensvariante, die über die vorherige Hydrolyse der Salze I und Zwischenisolierung der α-Ketocarbonsäure-N-acylamide V verläuft.

Das hier angegebene, über die erfindungsgemässen, neuen 5-Acyloxy-4(5H)-oxazoloniumsalze verlaufende Verfahren zur Herstellung von herbizid wirksamen asymmetrischen Triazionen IV ist dem vergleichbaren vorbekannten, über α-Ketocarbonsäure-N-tert.-butylamide verlaufenden Verfahren (vgl. DE-A Nrn. 2733180 und 2733181) technisch überlegen. Insbesondere können die erfindungsgemässen 5-Acyloxy-4(5H)-oxazoloniumsalze I mit Hydrazinderivaten VI, z.B. Thiocarbohydrazid oder S-Methylthiocarbohydrazid, unter sehr milden Bedingungen nahezu quantitativ zu asymmetrischen Triazinonen, die unmittelbar in hoher Reinheit anfallen, cyclisiert werden, während die vorbekannten α-Ketocarbonsäure-N-tert.-butylamide hierzu mit Thiocarbohydrazid mehrere Stunden auf 100°C erhitzt werden müssen und lediglich Ausbeuten von ca. 70% ergeben.

Bei der Durchführung des Verfahrens zur Herstellung von Triazinonen aus den erfindungsgemässen Salzen I setzt man im allgemeinen auf 1 mol eines Salzes der Formel I 1 bis 1,5 mol eines Hydrazinderivats der Formel VI ein. Die Reaktionstemperaturen liegen im allgemeinen zwischen 0 und 100, vorzugsweise zwischen 15 und 60°C.

Beispielsweise erhält man aus dem 5-Acetoxy-5-tert.-butyl-2-methyloxazoloniumsalz Ia nach folgendem Reaktionsschema die herbizid besonders wirksame Verbindung 4-Amino-6-tert.-butyl-3-methylthio-1,2,4-triazin-5(4H)-on IVb (vgl. DE-C Nr. 1795784 und US-A Nr. 3671523; ferner US-A Nr. 3905801 und DE-A Nr. 1695596).

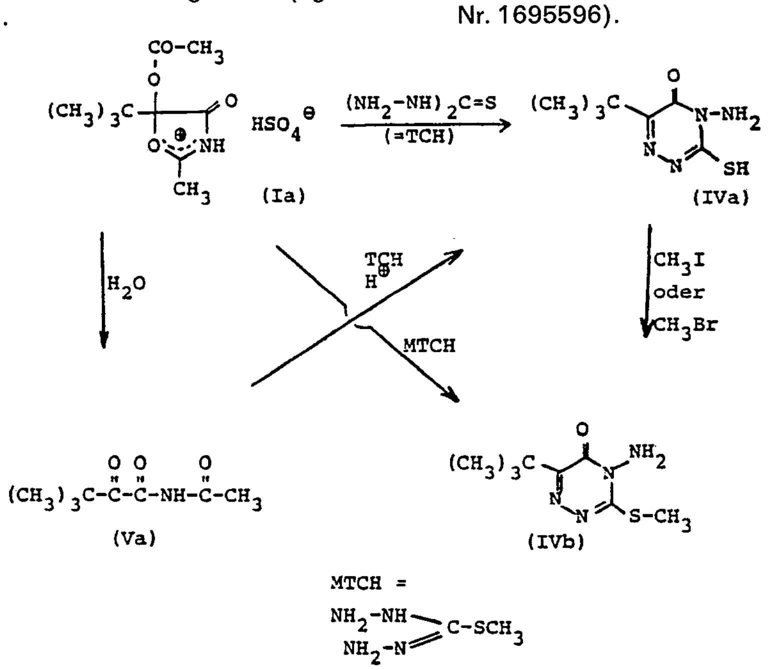

(Ia) (IVa) (Va) (IVb)

Die Methylierung IVa → IVb ist vorbekannt (vgl. z.B. „Chem. Ber.", *97*, S. 2173-8 [1964]; DE-A Nr. 2733180, ferner US-A Nrn. 3890317, 3897429 und 4035364).

Die nachfolgenden Herstellungsbeispiele sollen zur weiteren Erläuterung der Erfindung dienen.

*Herstellungsbeispiele*

*Beispiel 1*

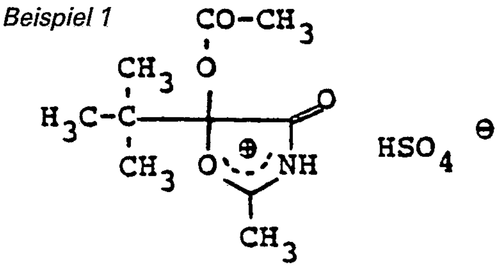

(Ia)

In 49,0 g (0,5 mol) vorgelegte konzentrierte Schwefelsäure werden jeweils bei Raumtemperatur zunächst 25,6 g (0,25 mol) Essigsäureanhydrid und anschliessend 27,8 g (0,25 mol) Pivaloylcyanid eingetragen. Nach 4stündigem Nachrühren wird die Reaktionsmischung spektroskopisch vermessen. Die charakteristischen Banden des IR-Spektrums sind in Tabelle 1 wiedergegeben, die Signale des $^1H$-NMR-Spektrums in Tabelle 2 und die Signale des $^{13}C$-NMR-Spektrums in Tabelle 3.

*Beispiel 2*

$(CH_3)_3C-CO-CO-NH-CO-CH_3$ (Va)

In 49,0 g (0,5 mol) vorgelegte konzentrierte Schwefelsäure werden jeweils bei Raumtemperatur zunächst 25,6 g (0,25 mol) Essigsäureanhydrid und anschliessend 27,8 g (0,25 mol) Pivaloylcyanid eingetragen. Nach 4stündigem Nachrühren wird die Reaktionsmischung mit 150 g Eiswasser versetzt und gut durchgerührt. Das ausfallende Reaktionsprodukt wird abgesaugt, mit 100 ml Wasser gewaschen und getrocknet. Man erhält 37,0 g (86% der Theorie) Trimethylbrenztraubensäure-N-acetylamid als farblose glänzende Blättchen vom Schmelzpunkt 82-84°C; Gehalt nach gaschromatographischer Bestimmung >98%.

*Beispiel 3*

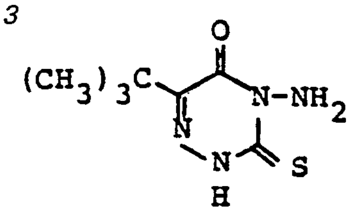

bzw.

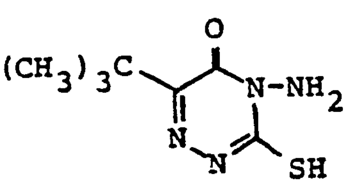

(IVa)

Man stellt analog zu Beispiel 1 die Lösung des 5-t.-Butyl-5-acetoxy-2-methyl-4(5H)-oxazoloniumhydrogensulfats her. Anschliessend wird diese Lösung in die Suspension von 29,3 g (0,275 mol) Thiocarbohydrazid in 300 ml Wasser getropft und anschliessend 1 h bei 50°C gerührt. Man kühlt und saugt den ausgefallenen Niederschlag ab und wäscht neutral. Nach Trocknen erhält man 48,0 g (95,9% der Theorie) 4-Amino-6-t.-butyl-3-mercapto-1,2,4-triazin-5(4H)-on, Schmelzpunkt 212-215°C.

*Beispiel 4*

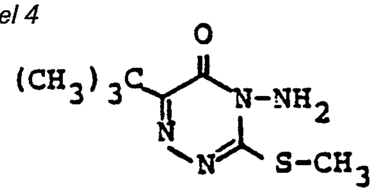

(IVb)

Man stellt analog zu Beispiel 1 die Lösung des Oxazoloniumhydrogensulfats Ia her. Diese Lösung wird in die Suspension von 74,4 g (0,3 mol) S-Methylthiocarbohydrazidhydroiodid in 300 ml Wasser getropft und anschliessend 0,5 h bei 50°C gerührt. Es scheidet sich ein Öl ab, das abgetrennt wird und 2mal mit 100 ml Wasser verrührt wird. Man erhält 39,9 g (74,6% der Theorie) rohes 4-Amino-6-t.-butyl-3-methylmercapto-1,2,4-triazin-5(4H)-on. Eine umkristallisierte Probe schmilzt bei 121-123°C.

**Patentansprüche**

1. 5-Acyloxy-4(5H)-oxazoloniumsalze der Formel (I):

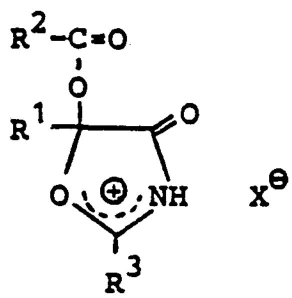

(I)

in welcher

$R^1$ für einen gegebenenfalls durch Alkoxy mit 1 bis 4 Kohlenstoffatomen, Carbalkoxy mit 1 bis 4 Kohlenstoffatomen in der Alkoxygruppe, Nitro, Cyano und/oder Halogen substituierten, geradkettigen oder verzweigten Alkylrest mit 1 bis 4 Kohlenstoffatomen; für einen gegebenenfalls durch Alkyl, Alkoxy oder Carbalkoxy mit jeweils bis zu 4 Kohlenstoffatomen, Nitro, Cyano und/oder Halogen substituierten Cycloalkylrest mit 3 bis 6 Kohlenstoffatomen im Ringsystem; für einen gegebenenfalls durch Alkyl, Alkoxy oder Carbalkoxy mit jeweils bis zu 4 Kohlenstoffatomen, Nitro und/oder Halogen substituierten Phenyl- oder Naphthylrest; oder für einen gegebenenfalls durch Alkyl, Alkoxy oder Carbalkoxy mit jeweils bis zu 4 Kohlenstoffatomen, Nitro, Cyano und/oder Halogen substituierten 5- oder 6gliedrigen heterocyclischen Rest steht, der 1 bis 3 Heteroatome wie Sauerstoff, Schwefel und/oder Stickstoff im Ring enthalten und ausserdem mit einem Benzolring annelliert sein kann,

$R^2$ und $R^3$ gleich oder verschieden sind und für einen gegebenenfalls chlorsubstituierten Alkylrest mit 1 bis 4 Kohlenstoffatomen oder für einen Phenylrest stehen, und

$X^{\ominus}$ für das Anion einer anorganischen oder organischen Säure mit einem $pK_s$-Wert kleiner als 2 steht,
in Form einer Lösung.

2. 5-Tertiärbutyl-5-acetoxy-2-methyl-4(5H)-oxazoloniumhydrogensulfat der Formel (Ia):

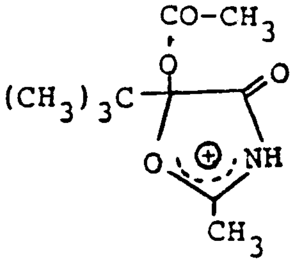

$HSO_4^{\ominus}$ (Ia)

in Form einer Lösung, gemäss Anspruch 1.

3. Verfahren zur Herstellung von 5-Acyloxy-4(5H)-oxazoloniumsalzen der Formel (I) gemäss Anspruch 1, dadurch gekennzeichnet, dass man Acylcyanide der allgemeinen Formel (II):

$$R^1-\overset{\overset{O}{\|}}{C}-CN \qquad (II)$$

in welcher
$R^1$ die in Anspruch 1 angegebene Bedeutung hat,
mit Carbonsäureanhydriden der allgemeinen Formel (III):

$$R^2-\overset{\overset{O}{\|}}{C}-O-\overset{\overset{O}{\|}}{C}-R^3 \qquad (III)$$

in welcher
$R^2$ und $R^3$ die in Anspruch 1 angegebene Bedeutung haben,
in Gegenwart einer anorganischen oder organischen Säure mit einem $pK_s$-Wert kleiner als 2 und gegebenenfalls in Gegenwart eines Lösungsmittels bei Temperaturen zwischen 0 und 120°C umsetzt.

4. Verfahren nach Anspruch 3, dadurch gekennzeichnet, dass man die Umsetzung bei Temperaturen zwischen 10 und 60°C durchführt.

5. Verfahren nach Anspruch 3, dadurch gekennzeichnet, dass man Acylcyanid (II) und Carbonsäureanhydrid (III) im Molverhältnis von 1:0,5-10 einsetzt.

6. Verfahren nach Anspruch 5, dadurch gekennzeichnet, dass man Acylcyanid (II) und Carbonsäureanhydrid (III) im Molverhältnis von 1:0,8-4 einsetzt.

7. Verfahren nach Anspruch 6, dadurch gekennzeichnet, dass man Acylcyanid (II) und Carbonsäureanhydrid (III) im Molverhältnis von 1:1-2 einsetzt.

8. Verfahren nach Anspruch 3, dadurch gekennzeichnet, dass man Acylcyanid (II) und Säure im Molverhältnis von 1:0,5-10 einsetzt.

9. Verfahren nach Anspruch 8, dadurch gekennzeichnet, dass man Acylcyanid (II) und Säure im Molverhältnis von 1:0,8-8 einsetzt.

10. Verfahren nach Anspruch 9, dadurch gekennzeichnet, dass man Acylcyanid und Säure im Molverhältnis von 1:1-4 einsetzt.

11. Verfahren nach Anspruch 3, dadurch gekennzeichnet, dass man Carbonsäureanhydrid (III) und Säure im Molverhältnis von 1:1-4 einsetzt.

12. Verfahren nach Anspruch 3, dadurch gekennzeichnet, dass man Acylcyanid (II), Carbonsäureanhydrid (III) und Säure im Molverhältnis 1:1:2 bis 1:2:4 einsetzt.

13. Verfahren nach Anspruch 3, dadurch gekennzeichnet, dass man als Acylcyanid (II) Pivaloylcyanid, als Carbonsäureanhydrid (III) Essigsäureanhydrid und als Säure konzentrierte Schwefelsäure einsetzt.

14. Verfahren zur Herstellung von 1,2,4-Triazin-5(4H)-onderivaten der Formel IV:

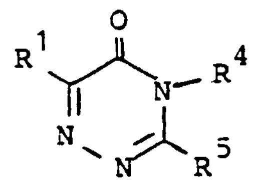

(IV)

in welcher
$R^1$ die oben angegebene Bedeutung hat,
$R^4$ für Wasserstoff, Alkyl mit 1 bis 4 C-Atomen, Aryl mit 6 bis 10 C-Atomen, Amino, mono- oder disubstituierte Aminogruppen steht, und
$R^5$ für Wasserstoff, Alkyl mit 1 bis 4 C-Atomen, Aryl mit 6 bis 10 C-Atomen, Hydroxy, Alkoxy mit 1 bis 4 C-Atomen, Aryloxy mit 6 bis 10 C-Atomen, Mercapto, Alkylmercapto mit 1 bis 4 C-Atomen, Amino, mono- oder disubstituierte Aminogruppen steht,
dadurch gekennzeichnet, dass man 5-Acyloxy-4(5H)-oxazoloniumsalze der Formel (I) gemäss Anspruch 1 mit Hydrazinderivaten der Formel (VI):

$$NH_2-N=\overset{\overset{R^5}{|}}{C}-NH-R^4 \qquad (VI)$$

in welcher
$R^4$ und $R^5$ die oben angegebene Bedeutung haben,
umsetzt.

## Claims

1. 5-Acyloxy-4(5H)-oxazolonium salts of the formula (I)

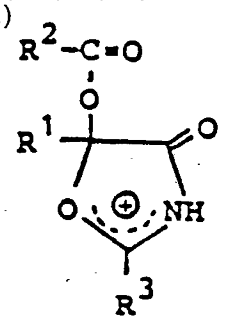

$X^{\ominus}$ (I)

in which
$R^1$ represents a straight-chain or branched alkyl radical which has 1 to 4 carbon atoms and is optionally substituted by alkoxy with 1 to 4 carbon atoms, carbalkoxy with 1 to 4 carbon atoms in the alkoxy group, nitro, cyano and/or halogen; a

cycloalkyl radical which has 3 to 6 carbon atoms in the ring system and is optionally substituted by alkyl, alkoxy or carbalkoxy with in each case up to 4 carbon atoms, nitro, cyano and/or halogen; a phenyl or naphthyl radical which is optionally substituted by alkyl, alkoxy or carbalkoxy with in each case up to 4 carbon atoms, nitro and/or halogen; or a 5- or 6-membered heterocyclic radical which is optionally substituted by alkyl, alkoxy or carbalkoxy with in each case up to 4 carbon atoms, nitro, cyano and/or halogen, can contain 1 to 3 hetero atoms such as oxygen, sulphur and/or nitrogen in the ring, and can furthermore be fused to a benzene ring,

$R^2$ and $R^3$ are identical or different and represent an optionally chlorine-substituted alkyl radical with 1 to 4 carbon atoms or a phenyl radical, and

$X^{\ominus}$ represents the anion of an inorganic or organic acid having a $pK_a$ value of less than 2, in the form of a solution.

2. 5-Tertiary-butyl-5-acetoxy-2-methyl-4-(5H)-oxazolonium hydrogen sulphate of the formula (Ia)

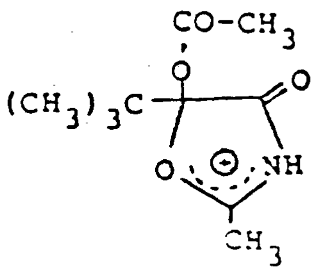

$HSO_4^{\ominus}$ (Ia)

in the form of a solution, according to Claim 1.

3. Process for the preparation of 5-acyloxy-4(5H)-oxazolonium salts of the formula (I) according to Claim 1, characterised in that acyl cyanides of the general formula (II)

$$R^1-\overset{\overset{O}{\|}}{C}-CN \quad (II)$$

in which

$R^1$ has the meaning given in Claim 1,

are reacted with carboxylic acid anhydrides of the general formula (III)

$$R^2-\overset{\overset{O}{\|}}{C}-O-\overset{\overset{O}{\|}}{C}-R^3 \quad (III)$$

in which

$R^2$ and $R^3$ have the meaning given in Claim 1,

in the presence of an inorganic or organic acid having a $pK_a$ value of less than 2, and, if appropriate, in the presence of a solvent, at temperatures between 0 and 120°C.

4. Process according to Claim 3, characterised in that the reaction is carried out at temperatures between 10 and 60°C.

5. Process according to Claim 3, characterised in that acyl cyanide (II) and carboxylic acid anhydride (III) are used in a molar ratio of 1:0.5-10.

6. Process according to Claim 5, characterised in that acyl cyanide (II) and carboxylic acid anhydride (III) are used in a molar ratio of 1:0.8-4.

7. Process according to Claim 6, characterised in that acyl cyanide (II) and carboxylic acid anhydride (III) are used in a molar ratio of 1:1-2.

8. Process according to Claim 3, characterised in that acyl cyanide (II) and the acid are used in a molar ratio of 1:0.5-10.

9. Process according to Claim 8, characterised in that acyl cyanide (II) and the acid are used in a molar ratio of 1:0.8-8.

10. Process according to Claim 9, characterised in that the acyl cyanide and the acid are used in a molar ratio of 1:1-4.

11. Process according to Claim 3, characterised in that carboxylic acid anhydride (III) and the acid are used in a molar ratio of 1:2.

12. Process according to Claim 3, characterised in that acyl cyanide (II), carboxylic acid anhydride (III) and the acid are used in a molar ratio of 1:1:2 to 1:2:4.

13. Process according to Claim 3, characterised in that pivaloyl cyanide is used as the acyl cyanide (II), acetic anhydride as the carboxylic acid anhydride (III) and concentrated sulphuric acid as the acid.

14. Process for the preparation of 1,2,4-triazin-5(4H)-one derivatives of the formula (IV)

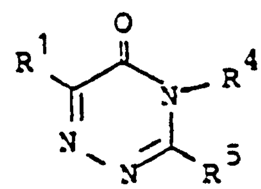

(IV)

in which

$R^1$ has the meaning given above,

$R^4$ represents hydrogen, alkyl with 1 to 4 C atoms, aryl with 6 to 10 C atoms, amino, or mono- or disubstituted amino groups, and

$R^5$ represents hydrogen, alkyl with 1 to 4 C atoms, aryl with 6 to 10 C atoms, hydroxyl, alkoxy with 1 to 4 C atoms, aryloxy with 6 to 10 C atoms, mercapto, alkylmercapto with 1 to 4 C atoms, amino, or mono- or disubstituted amino groups, characterised in that 5-acyloxy-4(5H)-oxazolonium salts of the formula (I) according to Claim 1 are reacted with hydrazine derivatives of the formula (VI)

$$NH_2-N=\overset{\overset{R^5}{|}}{C}-NH-R^4 \quad (VI)$$

in which

$R^4$ and $R^5$ have the meaning given above.

## Revendications

1. Sels de 5-acyloxy-4(5H)-oxazolonium de formule (I)

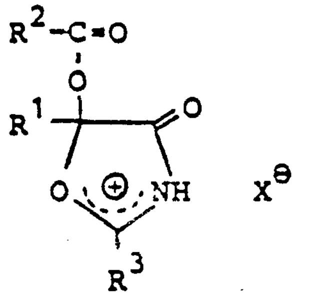

dans laquelle

$R^1$ représente un reste alkyle ayant 1 à 4 atomes de carbone à chaîne droite ou ramifiée, éventuellement substitué par un radical alcoxy ayant 1 à 4 atomes de carbone, carbalcoxy ayant 1 à 4 atomes de carbone dans le groupe alcoxy, nitro, cyano et/ou un halogène; un reste cycloalkyle ayant 3 à 6 atomes de carbone dans le noyau, éventuellement substitué par un radical alkyle, alcoxy ou carbalcoxy ayant chacun jusqu'à 4 atomes de carbone, nitro, cyano et/ou un halogène; un reste phényle ou naphtyle éventuellement substitué par un radical alkyle, alcoxy ou carbalcoxy ayant chacun jusqu'à 4 atomes de carbone, nitro et/ou un halogène; ou bien un reste hétérocyclique pentagonal ou hexagonal éventuellement substitué par un radical alkyle, alcoxy ou carbalcoxy ayant chacun jusqu'à 4 atomes de carbone, nitro, cyano et/ou un halogène, et pouvant contenir 1 à 3 hétéroatomes tels qu'oxygène, soufre et/ou azote dans le noyau et pouvant, en outre, être condensé avec un noyau benzénique,

$R^2$ et $R^3$ sont égaux ou différents et représentent un reste alkyle ayant 1 à 4 atomes de carbone éventuellement substitué par du chlore ou un reste phényle, et

$X^{\ominus}$ représente l'anion d'un acide inorganique ou organique ayant une valeur $pK_s$ inférieure à 2, sous la forme d'une solution.

2. Hydrogénosulfate de 5-tertio-butyl-5-acétoxy-2-méthyl-4(5H)-oxazolonium de formule (Ia)

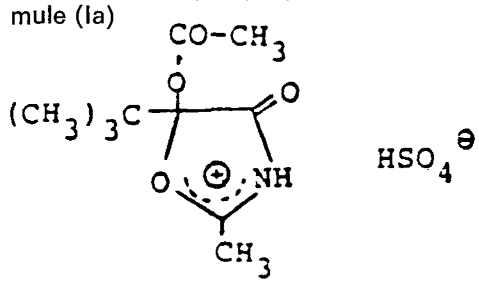

(Ia)

sous la forme d'une solution, suivant la revendication 1.

3. Procédé de production de sels de 5-acyloxy-4(5H)-oxazolonium de formule (I) suivant la revendication 1, caractérisé en ce qu'on fait réagir des cyanures d'acyle de formule générale (II)

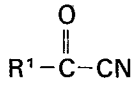

(II)

dans laquelle

$R^1$ a la définition indiquée dans la revendication 1,



avec des anhydrides d'acides carboxyliques de formule générale (III)

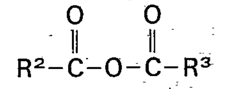

(III)

dans laquelle

$R^2$ et $R^3$ ont la définition indiquée dans la revendication 1,

en présence d'un acide inorganique ou organique ayant une valeur de $pK_s$ inférieure à 2 et, le cas échéant, en présence d'un solvant à des températures comprises entre 0 et 120° C.

4. Procédé suivant la revendication 3, caractérisé en ce qu'on conduit la réaction à des températures comprises entre 10 et 60° C.

5. Procédé suivant la revendication 3, caractérisé en ce qu'on utilise le cyanure d'acyle (II) et l'anhydride d'acide carboxylique (III) dans un rapport molaire de 1:0,5-10.

6. Procédé suivant la revendication 5, caractérisé en ce qu'on utilise le cyanure d'acyle (II) et l'anhydride d'acide carboxylique (III) dans un rapport molaire de 1:0,8-4.

7. Procédé suivant la revendication 6, caractérisé en ce qu'on utilise le cyanure d'acyle (II) et l'anhydride d'acide carboxylique (III) dans un rapport molaire de 1:1-2.

8. Procédé suivant la revendication 3, caractérisé en ce qu'on utilise le cyanure d'acyle (II) et l'acide dans un rapport molaire de 1:0,5-10.

9. Procédé suivant la revendication 8, caractérisé en ce qu'on utilise le cyanure d'acyle (II) et l'acide dans un rapport molaire de 1:0,8-8.

10. Procédé suivant la revendication 9, caractérisé en ce qu'on utilise le cyanure d'acyle et l'acide dans un rapport molaire de 1:1-4.

11. Procédé suivant la revendication 3, caractérisé en ce qu'on utilise l'anhydride d'acide carboxylique (III) et l'acide dans le rapport molaire de 1:2.

12. Procédé suivant la revendication 3, caractérisé en ce qu'on utilise le cyanure d'acyle (II), l'anhydride d'acide carboxylique (III) et l'acide dans la proportion molaire de 1:1:2 à 1:2:4.

13. Procédé suivant la revendication 3, caractérisé en ce qu'on utilise comme cyanure d'acyle (II) le cyanure de pivaloyle, comme anhydride d'acide carboxylique (III) l'anhydride d'acide acétique et comme acide l'acide sulfurique concentré.

14. Procédé de production de dérivés de 1,2,4-triazine-5(4H)-one de formule (IV)

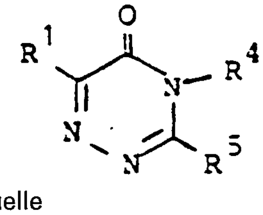

(IV)

dans laquelle

$R^1$ a la définition indiquée ci-dessus,

$R^4$ désigne l'hydrogène, un groupe alkyle ayant 1 à 4 atomes de carbone, aryle ayant 6 à 10 atomes de carbone, amino, des groupes amino monosubstitués ou disubstitués, et

$R^5$ désigne l'hydrogène, un groupe alkyle ayant 1 à 4 atomes de carbone, aryle ayant 6 à 10 atomes de carbone, hydroxy, alcoxy ayant 1 à 4 atomes de carbone, aryloxy ayant 6 à 10 atomes de carbone, mercapto, alkylmercapto ayant 1 à 4 atomes de carbone, amino, amino monosubstitué ou disubstitué,
caractérisé en ce qu'on fait réagir des sels de 5-acyloxy-4(5H)-oxazolonium de formule (I) suivant la revendication 1 avec des dérivés d'hydrazine de formule (VI)

$$NH_2-N=\overset{\displaystyle R^5 \atop |}{C}-NH-R^4 \qquad (VI)$$

dans laquelle
$R^4$ et $R^5$ ont la définition indiquée ci-dessus.